# EUROPÄISCHE PATENTSCHRIFT

(11) **EP 2 848 263 B1**
(45) Veröffentlichungstag und Bekanntmachung des Hinweises auf die Patenterteilung: **25.07.2018**
(21) Anmeldenummer: 14184487.8
(22) Anmeldetag: 11.09.2014
(51) Int. Cl.: A61L 2/08, B65B 55/08, B67C 7/00

(54) **Vorrichtung zum Sterilisieren von Kunststoffvorformlingen bei gleichzeitiger Innen- und Außensterilisierung**
Device for sterilising plastic parisons with simultaneous inner and external sterilisation
Dispositif de stérilisation des parisons de plastique à la stérilisation intérieure et externe simultanée

(30) Priorität: 11.09.2013 DE 102013109988
(43) Veröffentlichungstag der Anmeldung: 18.03.2015
(73) Patentinhaber: Krones AG, 93073 Neutraubling (DE)
(72) Erfinder: Söllner, Jürgen, 93073 Neutraubling (DE); Scheuren, Hans, 93073 Neutraubling (DE); Müller, Holger, 93073 Neutraubling (DE)
(74) Vertreter: Bittner, Bernhard

(56) Entgegenhaltungen:
- EP-A1- 1 982 921
- EP-A1- 2 601 975
- EP-A2- 2 505 505
- EP-A2- 2 594 493
- DE-A1-102011 055 552

## Beschreibung

Die vorliegende Erfindung bezieht sich auf eine Vorrichtung und ein Verfahren zum Sterilisieren von Kunststoffbehältnissen und insbesondere von Kunststoffvorformlingen. Aus dem Stand der Technik ist es bekannt, dass Kunststoffbehältnisse oder allgemein Behältnisse vor ihrer Befüllung sterilisiert werden. Zu dieser Sterilisation sind mehrere Verfahren bekannt, wie beispielsweise eine Sterilisation mittels Wasserstoffperoxid. In jüngerer Zeit ist man jedoch bestrebt, auf den Einsatz derartiger Chemikalien zu verzichten. Daher ist man teilweise auch dazu übergegangen, die Sterilisation durch Bestrahlung und insbesondere, aber nicht ausschließlich durch Bestrahlung mit Ladungsträgern wie beispielsweise Elektronen durchzuführen.

Im Stand der Technik laufen Kunststoffvorformlinge nach einer Erwärmung bzw. nach einem Ofen in eine Blasmaschine ein. Bei einem internen Stand der Technik der Anmelderin ist es bekannt, dass die Kunststoffvorformlinge nach dem Ofen in ein Sterilisationsmodul über einen Einlaufstern einlaufen, dann durch stationäre Emitter außen entkeimt werden und anschließend über eine Transporteinrichtung zu einem Innenentkeimungsmodul geführt werden, bei dem Finger in die Kunststoffvorformlinge eintauchen und diese innen sterilisieren. Anschließend werden die Kunststoffvorformlinge über einen Auslaufstern weiter transportiert. Bei der Außenentkeimung werden die Kunststoffvorformlinge an ihrer Innenseite über ein Greifelement gehaltert.

Auf diese Weise ist jedoch die Entkeimung der Kunststoffvorformlinge relativ aufwendig und setzt weiterhin auch eine Vielzahl von nacheinander angeordneten Modulen voraus.

So ist aus der EP 2 594 493 A2 eine Vorrichtung zum Sterilisieren zumindest eines Abschnitts einer Innenwandung und eines Abschnitts einer Außenwandung von Behältnissen. Eine zweite Sterilisationseinrichtung kann in einen zu sterilisierenden Kunststoffvorformling eingeführt werden. Bei der zweiten Sterilisationseinrichtung kann es sich um eine Ladungsträger emittierenden Einrichtung handeln.

In der EP 2 505 505 A2 wir eine Innensterilisationsvorrichtung von Kunststoffvorformlingen beschrieben. Hierzu ist eine Bestrahlungseinrichtung vorgesehen, die in eine über einen Haltedorn realisierte Halteeinrichtung der Vorformlinge integriert ist und mittels der ein in den Vorformlingen befindliches fließfähiges Medium mit Licht bestrahlt wird.

Die DE 10 2011 055 552 A1 beschreibt eine Außensterilisationseinrichtung für Kunststoffvorformlinge, bei der die Kunststoffvorformlinge an dieser vorbeigeführt werden. Gehalten werden die Kunststoffvorformlinge dabei an Halteelementen, die beispielsweise als an der Innenwand anliegenden Spannhülsen ausgebildet sind.

Aus der EP 1 982 921 A1 geht eine Sterilisierungseinrichtung, die Ladungsträger erzeugt, und deren Behandlungskopf in das Behältnis eingeführt werden kann hervor. Dabei werden Halteeinrichtungen für die zu sterilisierenden Behältnisse gezeigt, die das Behältnis umgreifen.

Der vorliegenden Erfindung liegt daher die Aufgabe zugrunde, eine Vorrichtung und ein Verfahren zur Verfügung zu stellen, welche den Aufwand zur Sterilisierung von Kunststoffvorformlingen reduzieren. Diese Aufgabe wird erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Sterilisieren von Kunststoffbehältnissen und insbesondere von Kunststoffvorformlingen weist eine Transporteinrichtung auf, welche die Kunststoffvorformlinge entlang eines vorgegebenen Transportpfades transportiert. Weiterhin weist die Vorrichtung eine erste Sterilisationseinrichtung auf, welche geeignet ist, eine Außenoberfläche der Kunststoffbehältnisse mit Strahlung zu beaufschlagen, um diese Außenoberfläche zu sterilisieren. Weiterhin weist die Vorrichtung eine zweite Sterilisationseinrichtung auf, welche geeignet ist, eine Innenoberfläche der Kunststoffbehältnisse mit Strahlung zu beaufschlagen, um diese Innenoberfläche zu sterilisieren.

Erfindungsgemäß ist an der Transporteinrichtung wenigstens eine Halteeinrichtung angeordnet, welche ein Eingriffselement aufweist, welches in einen Mündungsbereich des Kunststoffbehältnisses einführbar ist, um diesen zu halten. Dabei ist wenigstens ein Bestandteil der zweiten Sterilisationseinrichtung in diese Halteeinrichtung integriert. Es wird also vorgeschlagen, dass eine Halteeinrichtung vorgesehen ist, die neben der eigentlichen Haltefunktion auch eine Sterilisationsfunktion aufweist. Bevorzugt wird daher ein Sterilisationselement verwendet, welches bereits in eine Halteeinrichtung und insbesondere eine Innengreifeinrichtung für die Kunststoffvorformlinge integriert ist. Bevorzugt ist die Halteeinrichtung ein Innengreifer.

Bei der Strahlung handelt es sich um Teilchenstrahlung bzw. Ladungsträgerstrahlung und besonders bevorzugt um Elektronenstrahlung. Es könnten jedoch auch andere Strahlungsgattungen bzw. Ladungsträger verwendet werden, wie beispielsweise Protonen, Alphateilchen und dergleichen.

Bei einer weiteren vorteilhaften Ausführungsform weist die Vorrichtung eine Elektronenerzeugungseinrichtung auf und bevorzugt auch eine Beschleunigungseinrichtung, welche die Ladungsträger bzw. die Elektronen in einer vorgegebenen Beschleunigungsrichtung beschleunigt.

Bei einer weiteren vorteilhaften Ausführungsform weist die Vorrichtung auch ein Austrittsfenster auf, über welches die Ladungsträger austreten können und so beispielsweise auf eine Innenwandung der Behältnisse gelangen können.

Bei einer weiteren vorteilhaften Ausführungsform weist die Vorrichtung einen Sterilraum auf, innerhalb dessen die Kunststoffvorformlinge transportiert werden. Dabei ist bevorzugt weiterhin eine Dichtungseinrichtung vorgesehen, welche diesen Sterilraum gegenüber einer Umgebung abdichtet.

Vorteilhaft ist dieser Sterilraum kanalartig um einen Transportpfad der Kunststoffvorformlinge herum ausgebildet. Vorteilhaft weist die Vorrichtung auch Dichtungseinrichtungen auf, welche den Sterilraum abdichten und insbesondere Dichtungseinrichtungen, welche bezüglich einander bewegliche Wände gleichwohl abdichten. So kann bei einer möglichen Ausführungsform als Dichtungseinrichtung ein sogenanntes Wasserschloss verwendet werden, welches einen umlaufenden, mit Wasser gefüllten Kanal aufweist, in den ein Schwert des relativ beweglichen Bestandteils eintaucht.

Bei einer weiteren vorteilhaften Ausführungsform transportiert die Transporteinrichtung die Kunststoffbehältnisse wenigstens abschnittsweise entlang eines kreisförmigen Pfades. Vorteilhaft transportiert die Transporteinrichtung die Kunststoffbehältnisse wenigstens während deren Innensterilisation wenigstens abschnittsweise entlang eines kreisförmigen Pfades.

Bei einer weiteren vorteilhaften Ausführungsform ist eine Zuführeinrichtung vorgesehen, welche der Transporteinrichtung die Kunststoffvorformlinge zuführt. Vorteilhaft weist die Transporteinrichtung eine Vielzahl von Halteeinrichtungen zum Halten der Kunststoffbehältnisse auf.

Weiterhin sind vorteilhaft auch Bewegungseinrichtungen vorgesehen, welche eine Relativbewegung zwischen den Kunststoffbehältnissen und den Halteeinrichtungen in einer Längsrichtung der Kunststoffbehältnisse ermöglichen. Vorteilhaft werden dabei die Halteeinrichtungen relativ zu den Kunststoffbehältnissen bewegt. Bei diesen Bewegungseinrichtungen kann es sich beispielsweise um Elektromotoren und insbesondere um Linearmotoren handeln, welche den einzelnen Halteeinrichtungen zugeordnet sind, so dass eine individuelle Steuerung der Bewegungen der einzelnen Halteeinrichtungen möglich ist. Daneben wäre es jedoch auch möglich, die Bewegung der Halteeinrichtungen mittels Führungskurven zu realisieren. Die erste Sterilisationseinrichtung ist entlang des Transportpfades der Halteeinrichtung angeordnet, derart, dass die Kunststoffbehältnisse wenigstens zeitweise gleichzeitig von der ersten Sterilisationseinrichtung und von der zweiten Sterilisationseinrichtung sterilisiert werden. Im Gegensatz zum Stand der Technik kann daher die Vorrichtung mit lediglich einem Modul zur Durchführung einer Innen- und Außensterilisation auskommen.

Mit anderen Worten wird hier die Außen- und Innensterilisation bzw. Außen- und Innenentkeimung gleichzeitig durchgeführt, um so das Modul kompakter gestalten zu können. Dieses wird auch dadurch erreicht, dass die zweite Sterilisationseinrichtung in die Halteeinrichtung, beispielsweise den Preform-Innengreifer, der ansonsten ein Bestandteil der Außensterilisation ist, integriert wird.

Beim Ablauf des Verfahrens ist es denkbar, dass die Kunststoffvorformlinge beispielsweise seitlich in ihrem Gewinde bzw. Tragring etwa mit einem Einlaufstern transportiert werden. Bei dem Transferweg kurz vor dem Eingang der Außenentkeimung begleitet dieser Finger mit dem integrierten Emitter den Kunststoffvorformling vorzugsweise bereits mit. Das heißt die Halteeinrichtung befindet sich oberhalb der Kunststoffbehältnisse und kann somit bereits die Innenfläche der Kunststoffbehältnisse bzw. Kunststoffvorformlinge entkeimen.

Die Halteeinrichtung fährt dann während des Transports durch die Außenentkeimung in den Kunststoffvorformling ein und haltert diesen. Während dieser Zeit findet einerseits eine Bestrahlung der Außenoberfläche sowie auch der unteren Innenoberfläche der Kunststoffvorformlinge statt. Die obere Innenoberfläche ist aufgrund des Innengreifers bzw. der Halteeinrichtung in diesem Zeitraum nicht erreichbar. Beim Auslauf aus der Außenentkeimung fährt jedoch die Halteeinrichtung wieder aus dem Kunststoffvorformling heraus (sobald die Halteeinrichtung am Auslaufstern den Kunststoffvorformling von außen haltert) und begleitet den Kunststoffvorformling wieder, wodurch wieder die gesamte (obere und untere) Innenoberfläche des Kunststoffvorformlings entkeimt wird.

Vorteilhaft wird daher während der Sterilisation des unteren Bereiches des Kunststoffvorformlings die Halteeinrichtung nicht gegenüber dem Kunststoffvorformling bewegt, sondern die Halteeinrichtung wird bevorzugt nur bis zu einem gewissen Maß, insbesondere in einen Mündungsbereich des Kunststoffvorformlings eingeführt.

Durch diese Vorgehensweise kann auch der Weg, den das Kunststoffbehältnis während seiner Sterilisation durchläuft, verkürzt werden. Im Stand der Technik entsteht durch den relativ langen Weg der Kunststoffvorformlinge durch das Modul und die nicht gleichzeitig stattfindende Innen- und Außenentkeimung ein Rekontaminationspotential der bereits behandelten Kunststoffvorformlinge. Weiterhin kühlen die Kunststoffvorformlinge durch den vergleichsweise langen Laufweg ab, wobei andererseits eine bestimmte Zeit nicht überschritten werden darf, da sonst das durch den vorher angeordneten Ofen aufgebrachte Temperaturprofil verwischt wird und die Kunststoffvorformlinge wieder auskühlen und damit schlechter bzw. gar nicht mehr geblasen werden können.

Bei einer weiteren vorteilhaften Ausführungsform weist die Vorrichtung eine Zuführeinrichtung auf, welche der Halteeinrichtung die Kunststoffbehältnisse zuführt und diese Zuführeinrichtung oder die Transporteinrichtung sind derart ausgeführt, dass sie einen Abstand zwischen zwei entlang des Transportpfades benachbarten Kunststoffbehältnissen verändern können. Vorteilhaft ist die Zuführeinrichtung in dieser Weise ausgeführt. Es wird daher insbesondere vorgeschlagen, die Zuführung über einen sogenannten Verzugsstern durchzuführen, der die Teilung zwischen den einzelnen Kunststoffvorformlingen verzögern kann.

Vorteilhaft transportiert die Zuführeinrichtung die Kunststoffvorformlinge in diesem Bereich auch nicht entlang einer genau kreisförmigen Bahn, sondern es ist denkbar, dass die Kunststoffvorformlinge in dem Übergabebereich einen bestimmten Wegabschnitt mit den Halteeinrichtungen der Sterilisationseinrichtung mitbewegt werden. Mit anderen Worten wird, um den durch den Stirngreifer bedingten Behandlungsschatten zu kompensieren, die Übergabe in das und/oder aus dem Behandlungskarussell in die Länge gezogen.

Der erwähnte Teilungsverzugsstern, der die Kunststoffbehältnisse an das Behandlungskarussell übergibt, bzw. dessen Haltemittel begleiten bei der Übergabe die Kunststoffbehältnisse und die Halteeinrichtung bzw. der Stirngreifer bleibt bis zum letzten Moment kurvengesteuert oben bzw. von der Mündung des Kunststoffbehältnisses beabstandet. In diesem Zeitraum kann die zweite Sterilisationseinrichtung bereits permanent strahlen und so den inneren Mündungsbereich der Kunststoffvorformlinge entkeimen. Beim Ausfahren aus dem Behandlungskarussell werden die Kunststoffvorformlinge sofort an einen weiteren Teilungsverzugsstern übergeben, die Halteeinrichtung fährt nach oben und der nunmehr wieder freigegebene Mündungsbereich wird ein zweites Mal durch das Begleiten des Teilungsverzugssterns am Behandlungskarussell behandelt.

Durch diese Vorgehensweise kann das Volumen des gesamten Moduls wesentlich kleiner werden und damit ist auch weniger Strahlenschutzaufwand nötig, was zu einer Materialeinsparung führt. Weiterhin wird auch, wie oben erwähnt, das Risiko einer Rekontamination reduziert. Das gesamte Modul kann auf diese Weise um zwei Transportsterne reduziert werden und damit vermindern sich auch die mechanisch aufwendigen Übergaben und das ganze System wird prozessstabiler. Durch die kürzere Durchlaufzeit durch das Modul verschwindet auch die Problematik der Ausgleichszeit (der Kunststoffvorformling muss nach einer kurzen Zeit nach der Erhitzung geblasen werden). Weiterhin ist durch den einfacheren Aufbau des Moduls die zielgerichtete Führung der notwendigen Lüftungstechnik einfacher zu realisieren. Auch wird zur Aufrechterhaltung eines Überdrucks gegen eine Umgebung weniger Sterilluft benötigt.

Bei einer weiteren Ausführungsform transportiert damit die Transporteinrichtung und/oder die Zuführeinrichtung die Kunststoffbehältnisse entlang Abschnitten des Transportpfades, welche unterschiedliche Krümmungsradien aufweisen. Die gleiche Vorgehensweise ist auch im Bereich der Abführeinrichtung von der Transporteinrichtung denkbar.

Bei einer weiteren vorteilhaften Ausführungsform weist die Zuführeinrichtung wenigstens eine zweite Halteeinrichtung auf, welche geeignet ist, das Kunststoffbehältnis an einem Außenbereich zu greifen. Insbesondere kann es sich um Greifklammern handeln, welche die Kunststoffbehältnisse unterhalb eines Tragrings greifen, insbesondere um ansteuerbare Klammern.

Die vorliegende Erfindung ist weiterhin auf eine Halteeinrichtung zum Halten von Kunststoffbehältnissen gerichtet. Diese Halteeinrichtung weist ein Eingriffselement auf, welches in einem Mündungsbereich des Kunststoffbehältnisses einführbar ist, um diesen zu halten.

Erfindungsgemäß ist in diese Halteeinrichtung eine Sterilisationseinrichtung integriert, wobei diese Sterilisationseinrichtung ein Austrittsfenster aufweist, durch welches Ladungsträger austreten können, um einen Innenwandungsbereich der Kunststoffbehältnisse zu sterilisieren. Es wird also im Rahmen der Erfindung auch eine spezielle Halteeinrichtung, insbesondere ein Haltedorn, vorgeschlagen, der auch neben der Haltefunktion über eine Möglichkeit verfügt, eine Sterilisation, insbesondere einer Innenwandung eines Kunststoffbehältnisses und insbesondere eines Kunststoffvorformlings, durchzuführen. Im Stand der Technik werden üblicherweise derartige Sterilisationseinrichtungen in der Art eines Strahlfingers in das Behältnis eingeführt, das heißt beispielsweise auch während der Sterilisation gegenüber dem Kunststoffbehältnis bewegt.

Bei der vorgeschlagenen Variante wird bevorzugt die Halteeinrichtung zumindest zeitweise nicht gegenüber dem Kunststoffvorformling bewegt, sondern hält diesen insbesondere an seinem Mündungsbereich. Dies eignet sich insbesondere bei der Sterilisation von Kunststoffvorformlingen.

Bei einer weiteren vorteilhaften Ausführungsform weist die Halteeinrichtung auch eine Kühleinrichtung auf, um das besagte Austrittsfenster zu kühlen. Dabei ist es insbesondere vorgesehen, dass ein Luftstrom an das Austrittsfenster herangeführt wird. Vorteilhaft wird dabei eine Sterilluft an das Austrittsfenster herangeführt. Dabei können beispielsweise Kühlluftkanäle vorgesehen sein, welche sich auch in einer Längsrichtung der Halteeinrichtung erstrecken. Vorteilhaft weist die Halteeinrichtung ein Gehäuse bzw. einen abgeschlossenen Raum auf, innerhalb dessen die Elektronen sich bewegen, um schließlich durch das Austrittsfenster auszutreten. Vorteilhaft ist innerhalb dieses Gehäuses ein Vakuum bzw. ein Teilvakuum ausgebildet. Daneben weisen bevorzugt die einzelnen Sterilisationseinrichtungen jeweils auch Ladungsträgererzeugungseinrichtungen auf und bevorzugt auch Beschleunigungseinrichtungen, welche die erzeugten Ladungsträger, insbesondere Elektronen, in Richtung des Austrittsfensters beschleunigen.

Daneben wäre es auch denkbar und bevorzugt, dass die Strahlungseinrichtungen bzw. Fingeremitter die (Kühl)Luft durch eine Doppelwandung führen. So kann beispielsweise um das Gehäuse des Strahlfingers ein weiteres Rohr angeordnet sein, bevorzugt ein Titan-Überwurfrohr. Zwischen diesen Rohren kann die Kühlluft zum Austrittsfenster gelangen und dieses kühlen.

Daneben wäre es jedoch auch möglich, die Kühlluft zumindest abschnittsweise über die Halteeinrichtungen zu führen. Diese Halteeinrichtungen können beispielsweise Kanäle zum Leiten der Kühlluft aufweisen. Auch könnte die Kühlluft zwischen Bestandteilen der Halteeinrichtung geführt werden, etwa zwischen zwei benachbarten Vorsprüngen der Halteeinrichtung. Zusätzlich oder alternativ wäre auch die Verwendung einer Flüssigkeitskühlung denkbar.

Vorteilhaft ist die Kühleinrichtung derart ausgebildet, dass sie das Austrittsfenster von außerhalb mit dem Kühlmittel, beispielsweise mit Luft, beaufschlagt. Es wären jedoch auch (zusätzlich oder alternativ) andere Kühleinrichtungen denkbar, wie beispielsweise Flüssigkeitskühlungen.

Bei einer weiteren vorteilhaften Ausführungsform ist das besagte Gehäuse, innerhalb dessen die Ladungsträger zu dem Austrittsfenster gelangen, wenigstens abschnittsweise von dem Eingriffselement umgeben. Auf diese Weise ist eine sehr kompakte Gestaltung der Halteeinrichtung denkbar.

Bei einer vorteilhaften Ausführungsform weist das Eingriffselement eine Vielzahl von Eingriffsabschnitten auf, welche auf die Mündung zuspannbar sind. Vorteilhaft können dabei zwischen den einzelnen Eingriffsabschnitten jeweils Spalten vorgesehen sein, welche sich bevorzugt auch in einer Längsrichtung der Halteeinrichtung erstrecken.

Bei einer weiteren vorteilhaften Ausführungsform steht das Austrittsfenster in einer Längsrichtung der Halteeinrichtung gegenüber dem Eingriffselement hervor. Dieses bedeutet, dass das Austrittsfenster in Richtung des Kunststoffvorformlings bzw. dessen Bodenbereichs gegenüber dem Eingriffselement hervorsteht. Auf diese Weise wird erreicht, dass die austretenden Ladungsträger nicht durch Bereiche des Eingriffselements an der Sterilisation der Kunststoffvorformlinge gehindert werden. So kann eine Sterilisation in alle Umfangsrichtungen erfolgen.
Bei einer weiteren bevorzugten Ausführungsform weist die Vorrichtung eine Unterdruckerzeugungseinrichtung auf, welche das Kunststoffbehältnis während dessen Sterilisation wenigstens zeitweise mit einem Unterdruck beaufschlagt. Diese Vorgehensweise eignet sich insbesondere für die Sterilisation von Kunststoffvorformlingen, da diese eine relativ hohe Wanddicke aufweisen und daher mit einem Unterdruck beaufschlagbar sind, ohne sich dabei zu verformen.

Daneben könnte die Unterdruckbeaufschlagungseinrichtung derart ausgebildet sein, dass sie Luft aus dem Kunststoffbehältnis heraussaugt und diese Luft wiederum zumindest teilweise zum Kühlen des Austrittsfensters verwendet wird. So wäre es denkbar, einen bestimmten Anteil der abgesaugten Luft wieder zum Kühlen des (selben) Austrittsfensters zu verwenden.

Daneben könnte auch die Luft, die aus einem bestimmten Kunststoffvorformling abgesaugt wird, zur Kühlung eines weiteren Kunststoffvorformlings verwendet werden.

Die vorliegende Erfindung ist weiterhin auf ein Verfahren zum Sterilisieren von Kunststoffbehältnissen und insbesondere von Kunststoffvorformlingen gerichtet. Dabei werden die Kunststoffvorformlinge mittels einer Transporteinrichtung entlang eines vorgegebenen Transportpfades transportiert und während dieses Transports wird wenigstens eine Außenoberfläche der Kunststoffvorformlinge von einer ersten Sterilisationseinrichtung mit einer diese Außenoberfläche sterilisierenden Strahlung beaufschlagt.

Erfindungsgemäß werden die Kunststoffbehältnisse mittels Halteeinrichtungen transportiert, welche in einem Mündungsbereich der Kunststoffbehältnisse in diese eingeführt werden, wobei wenigstens zeitweise (insbesondere während des Transports der Kunststoffvorformlinge mittels der Transporteinrichtung) eine Innenoberfläche der Kunststoffbehältnisse von einer zweiten Sterilisationseinrichtung mit einer dieser Innenoberfläche sterilisierenden Strahlung beaufschlagt wird und wenigstens zeitweise die Beaufschlagung der Innenoberfläche und die Beaufschlagung der Außenoberfläche gleichzeitig erfolgen.

Vorteilhaft handelt es sich bei der Strahlung um eine Ladungsträgerstrahlung und insbesondere um eine Elektronenstrahlung. Damit werden zumindest zeitweise die Behältnisse sowohl von innen her als auch von außen her bestrahlt.

Vorteilhaft ist, wie oben erwähnt, eine Sterilisationseinrichtung in die Halteeinrichtung integriert, wobei diese Strahlungseinrichtung bzw. die Sterilisationseinrichtung die Innenoberfläche der Kunststoffbehältnisse von innen her beaufschlagt.

Bei einem weiteren vorteilhaften Verfahren werden die Behältnisse mittels einer Zuführeinrichtung der Transporteinrichtung zugeführt. Vorteilhaft werden dabei die Kunststoffbehältnisse entlang eines Transportpfades zugeführt, der teilweise kreisförmig verläuft, teilweise jedoch auch nicht kreisförmig verläuft. So kann insbesondere in einem Übergabebereich vorgesehen sein, dass die Kunststoffbehältnisse beispielsweise geradlinig gefördert werden oder entlang sich ändernder Krümmungsradien.

Bei einer weiteren vorteilhaften Ausführungsform greift die Zuführeinrichtung die Kunststoffbehältnisse von außen her und es werden wenigstens zeitweise während dieses Transports die Halteeinrichtungen mitgeführt. Auch während der Abführung von der Transporteinrichtung können die Kunststoffvorformlinge entlang oder mittels unterschiedlicher Krümmungsradien transportiert werden. Vorteilhaft werden innere Mündungsbereiche des Kunststoffvorformlings zeitlich vor und/oder nach der Sterilisation der Außenbereiche sterilisiert.

Bei einem weiteren vorteilhaften Verfahren werden die Kunststoffbehältnisse zumindest zeitweise während ihrer Sterilisation innerhalb eines Reinraums transportiert.

Bei einem weiteren vorteilhaften Verfahren wird während einer Zustellbewegung der Halteeinrichtung auf die Kunststoffbehältnisse zu und/oder während einer Wegführbewegung der Halteeinrichtung von den Kunststoffbehältnissen weg eine Sterilisation des inneren Mündungsbereiches und insbesondere des im Wesentlichen gesamten inneren Mündungsbereichs der Kunststoffbehältnisse und/oder eines oberen Mündungsbereichs vorgenommen.

Unter dem oberen Mündungsbereich wird dabei insbesondere derjenige Abschnitt verstanden, der sich direkt angrenzend an den Mündungsrand des Kunststoffbehältnisses befindet. Bevorzugt handelt es dabei um einen Abschnitt, der in dem Bereich eines (Außen)gewindes des Kunststoffbehältnisses liegt und/oder einem Bereich zwischen dem Außengewinde und einem Mündungsrand (bezogen auf eine Längsrichtung des Kunststoffbehältnisses, welche sich von der Mündung des Behältnisses zu einem Boden, insbesondere einer Bodenkuppe erstreckt).

Bei einer weiteren vorteilhaften Ausführungform ist die Vorrichtung zur Sterilisation der Kunststoffbehältnisse nach einer Erwärmungseinrichtung und/oder vor dem Ende einer Umformmaschine angeordnet ist. Bei dieser Umformmaschine kann es sich insbesondere um eine Blasformmaschine und insbesondere um eine Streckblasmaschine bzw. dessen Blasrad handeln.

Vorteilhaft wird dabei eine Sterilisation dieses inneren Mündungsbereiches mittels Strahlung und insbesondere mittels Ladungsträger-Strahlung vorgenommen. Bevorzugt werden bei der Zustell- und/oder Wegführbewegung auch die oberen Außenflächen der Kunststoffbehältnisse sterilisiert. So kann insbesondere das Gewinde des Kunststoffbehältnisses bzw. Kunststoffvorformlings sterilisiert werden. Bei einem weiteren vorteilhaften Verfahren werden die Halteeinrichtungen in der Längsrichtung der Kunststoffbehältnisse auf diese zugestellt. Vorteilhaft findet eine Sterilisation der direkten inneren Mündungsbereiche der Kunststoffvorformlinge während einer Zuführung an die Transporteinrichtung und/oder während einer Abführung von der Transporteinrichtung statt.

Bevorzugt werden bei der Sterilisation des Kunststoffbehältnisses bzw. Kunststoffvorformlings die folgenden Schritte durchgeführt, wobei darauf hingewiesen wird, dass einige der genannten Schritte auf weggelassen werden können:
- Das Kunststoffbehältnis wird unter den Fingerstrahler bzw. die (zweite) Sterilisationseinrichtung geführt. Das Kunststoffbehältnis wird im Mündungsbereich entkeimt;
- Die Halteeinrichtung fährt in das Kunststoffbehältnis;
- Die zweite Sterilisationseinrichtung, insbesondere ein Strahlfinger fährt weiter in das Kunststoffbehältnis ein (was jedoch insbesondere durch eine Bewegung des Kunststoffbehältnisses erfolgt); in diesem Schritt können bevorzugt bereits Ladungsträger bzw. Elektronen von der Sterilisationseinrichtung abgegeben werden, um die Innenwandung des Kunststoffbehältnisses zu sterilisieren. Vorzugsweise wird jedoch, wie gesagt, der Strahlfinger hier nicht bewegt sondern umgekehrt das Kunststoffbehältnis mit der Halteeinrichtung auf den Strahlfinger zu bewegt;
- Die zweite Sterilisationseinrichtung, insbesondere ein Strahlfinger fährt bis in einen Bodenbereich des Kunststoffbehältnisses ein; in diesem Schritt können bevorzugt bereits Ladungsträger bzw. Elektronen von der Sterilisationseinrichtung abgegeben werden, um die Innenwandung des Kunststoffbehältnisses zu sterilisieren.
- Das Kunststoffbehältnis und die Halteeinrichtung werden heruntergefahren und damit die Sterilisationseinrichtung aus dem Kunststoffbehältnis herausgezogen. Anschließend wird das Kunststoffbehältnis auf einen Außengreifer übergeben und die entkeimte Preform weiterverfahren; auch während dieses Zurückziehens der Sterilisationseinrichtung bzw. dem Herunterfahren des Kunststoffbehältnisses kann nochmals eine Sterilisation des (insbesondere äußeren) Mündungsbereichs des Kunststoffbehältnisses stattfinden.

Weitere Vorteile und Ausführungsformen ergeben sich aus den beigefügten Zeichnungen. Darin zeigen:
- Fig. 1: Eine Sterilisationsanordnung nach dem internen Stand der Technik der Anmelderin;
- Fig. 2: eine schematische Darstellung einer Sterilisationseinrichtung gemäß der Erfindung;
- Fig. 3: eine schematische Darstellung einer Halteeinrichtung mit integrierter Sterilisationseinrichtung;
- Fig. 4: eine Schnittdarstellung einer in einem Kunststoffvorformling eingeführten Sterilisationseinrichtung; und
- Fig. 5: einen Ablauf einer Behältnissterilisation.

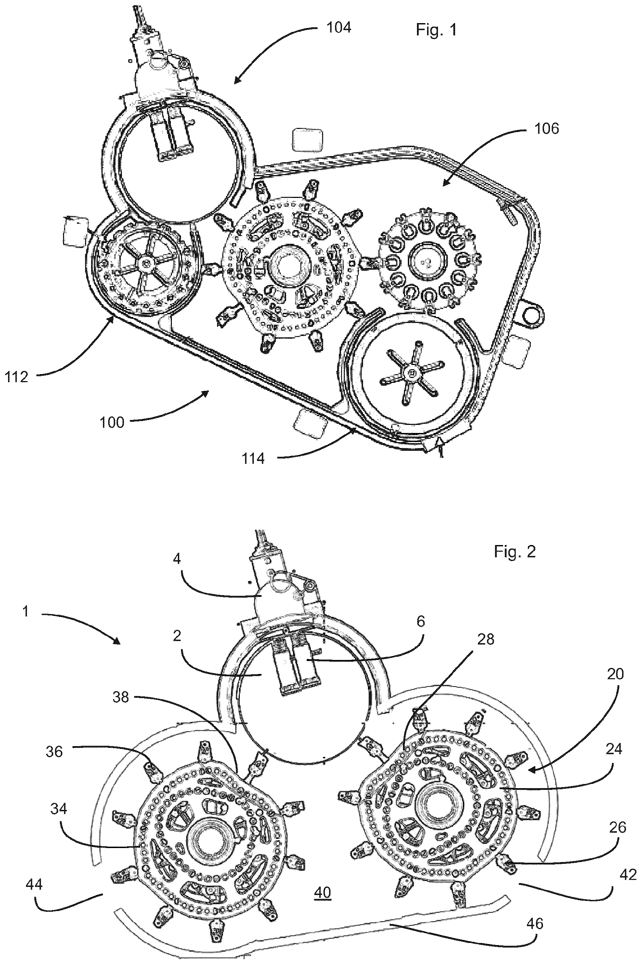

Fig. 1 zeigt eine Vorrichtung 100 zum Sterilisieren von Behältnissen nach dem internen Stand der Technik der Anmelderin. Dabei ist zunächst eine Zuführeinrichtung 112 vorgesehen, welche die Kunststoffvorformlinge einer Außensterilisationseinheit 104 zuführt. Ausgehend von dieser Außensterilisationseinheit 104 werden die Kunststoffbehältnisse abgenommen und zu einer Innensterilisationseinheit 106 geführt. Schließlich werden die Kunststoffbehältnisse über eine Abführeinrichtung 114 wieder abgeführt. Auf diese Weise sind insgesamt zwei Zuführeinheiten 112, 114 sowie eine weitere Transfereinheit zwischen den beiden Sterilisationseinrichtungen 104 und 106 erforderlich.

Fig. 2 zeigt eine schematische Darstellung einer erfindungsgemäßen Vorrichtung 1 zum Sterilisieren von Kunststoffvorformlingen. Hierbei werden zunächst über eine Zuführöffnung 44 die Kunststoffvorformlinge an eine Zuführeinrichtung 34 zugeführt. Diese Zuführeinrichtung 34 weist dabei eine Vielzahl von Halteeinrichtungen auf, welche die Kunststoffvorformlinge halten, beispielsweise an deren Außenumfang, beispielsweise unterhalb deren Tragring.

Weiterhin weist die Zuführeinrichtung 34 einen Übergabebereich 38 auf, in dem die Halteeinrichtungen und damit auch die an diesen angeordneten Kunststoffvorformlinge bevorzugt nicht entlang eines kreisförmigen Transportpfades geführt werden, sondern mittels unterschiedlicher Krümmungsradien, sodass der Übergabebereich an die Transporteinrichtung 2 verlängert werden kann. Die Transporteinrichtung 2 kann hier als drehbares Rad ausgeführt sein.

Das Bezugszeichen 4 kennzeichnet eine erste Sterilisationseinrichtung, welche zur Außensterilisation der Kunststoffvorformlinge dient. Dabei könnte diese erste Sterilisationseinrichtung beispielsweise entlang eines Transportpfades der Kunststoffvorformlinge stationär angeordnet sein.

Das Bezugszeichen 20 kennzeichnet eine Abführeinrichtung, welche zum Abführen der Kunststoffvorformlinge aus der Vorrichtung 1 dient. Auch diese weist wiederum einen Träger 24 auf, an dem eine Vielzahl von Halteeinrichtungen 26 angeordnet ist. Auch diese werden wiederum abschnittsweise (in dem Übergabebereich 28) entlang eines nicht kreisförmigen Transportpfades transportiert.

Das Bezugszeichen 42 kennzeichnet eine weitere Ausführöffnung, über welche die nunmehr sterilisierten Kunststoffvorformlinge aus der Vorrichtung ausgeführt werden.

Das Bezugszeichen 40 kennzeichnet einen Reinraum, innerhalb dessen die Sterilisation und auch der Transport der Kunststoffvorformlinge erfolgen. Dieser Reinraum wird mittels Wandungen 46 gegenüber einer (unsterilen) Umgebung abgedichtet. Innerhalb dieses Reinraums könnten auch Sterilisationseinrichtungen und/oder Reinigungseinrichtungen angeordnet sein, welche Bestandteile der Vorrichtung, wie etwa die Halteeinrichtungen 36, 26 zu deren Reinigung und/oder Sterilisation mit einem fließfähigen Sterilisations- und/oder Reinigungsmittel (beispielsweise Wasserstoffperoxid oder Peressigsäure) beaufschlagen. Auf diese Weise könnte auch eine sog. CIP - Reinigung der Anlage ermöglicht werden. Es könnte jedoch auch eine Sterilisation von Anlagenteilen mittels elektromagnetischer und/oder Elektronenstrahlung erfolgen.

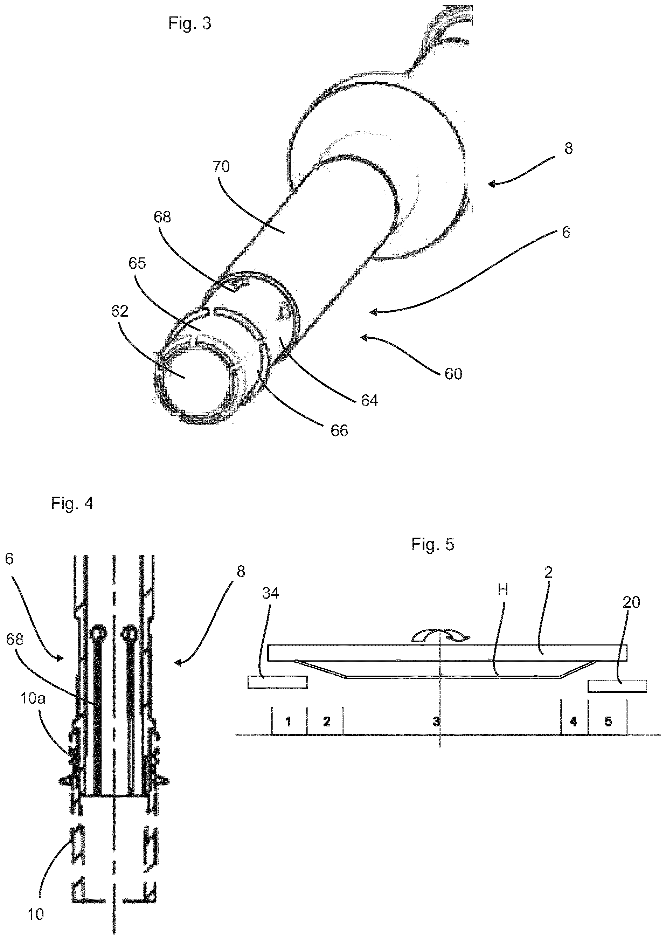

Fig. 3 zeigt eine Ausgestaltung einer zweiten Sterilisationseinrichtung, welche in eine Halteeinrichtung 8 integriert ist. Das Bezugszeichen 70 kennzeichnet dabei einen Grundkörper dieser Halteeinrichtung und die Bezugszeichen 64 eine Vielzahl von Halteelementen bzw. Eingriffsabschnitten, welche sich nach außen gegen eine Innenwandung des Kunststoffvorformlinge anlegen können, um diese zu halten. Das Bezugszeichen 68 kennzeichnet Schlitze, die zwischen den einzelnen Halteelementen angeordnet sind. Das Bezugszeichen 65 kennzeichnet einen zweiten Gehäuseabschnitt, innerhalb dessen die Elektronen bewegbar sind. Das Bezugszeichen 62 kennzeichnet das Austrittsfenster, über welches die Ladungsträger bzw. Elektronen austreten können.

Fig. 4 zeigt eine Darstellung, bei der die Halteeinrichtung 8 gerade in einen Kunststoffvorformling 10 eingesetzt ist. Das Bezugszeichen 10a kennzeichnet dabei einen Mündungsbereich dieses Kunststoffvorformlings. Auch sind wieder die Schlitze 68 zwischen den einzelnen Halteelementen erkennbar.

Fig. 5 zeigt eine Veranschaulichung des Verlaufes der Sterilisation. Dabei sind schematisch die Zuführeinrichtung 34 und die Abführeinrichtung 20 dargestellt. Das Bezugszeichen 2 kennzeichnet wiederum die Transporteinrichtung, die hier auch in einer Abwicklung dargestellt ist. Das Bezugszeichen H kennzeichnet eine Hubkurve der Halteeinrichtung, welche die Kunststoffvorformlinge während ihres Transportes mit der zweiten Transporteinrichtung 2 hält. Man erkennt, dass in einen Bereich 1 die Halteeinrichtung 36 der Zuführeinrichtung 34 die Halteeinrichtung 8 begleitet, da in diesem Bereich die beiden schematisch dargestellten Einrichtungen 34, 2 überschnitten dargestellt sind. In diesem Bereich 1 kann eine Sterilisation des Mündungsbereiches des Kunststoffvorformlings, insbesondere durch die zweite Sterilisationseinrichtung, stattfinden.

In dem Bereich 2 fährt die Halteeinrichtung 8 bzw. der Stirngreifer in den Kunststoffvorformling ein und die Halteeinrichtung 36 der Zuführeinrichtung 34 wird abgezogen. In diesem Zeitabschnitt kann auch bereits eine Innensterilisation eines unteren Bereichs des Kunststoffvorformlings stattfinden.

In dem Bereich 3 findet auch eine Außenbehandlung des Kunststoffvorformlings durch die erste Sterilisationseinrichtung statt. Daneben kann in diesem Bereich auch eine Innensterilisation des unteren Bereiches des Kunststoffvorformlings stattfinden.

In dem Bereich 4 greift die Halteeinrichtung der Abführeinrichtung 20 den Kunststoffvorformling und die Halteeinrichtung 8 wird, wie durch die Hubkurve H veranschaulicht, nach oben abgezogen. Auch in diesem Bereich wird der Kunststoffvorformling ein zweites Mal in seinem Mündungsbereich, insbesondere im oberen Mündungsbereich, behandelt.

In dem Bereich 5 begleiten, ähnlich wie in dem Bereich 1, sowohl die Halteeinrichtung der Abführeinrichtung den Kunststoffvorformling als auch die Halteeinrichtung 8 der Transporteinrichtung 2. Auch hier wird damit ein zweites Mal ein Mündungsbereich des Kunststoffvorformlings sterilisiert.

Wie ausgeführt, sind bevorzugt die Zuführeinrichtung 34 und/oder die Abführeinrichtung 20 jeweils als sog. Teilungsverzugssterne ausgeführt. Dabei ist es möglich, dass die einzelnen Halteelemente dieser Zuführeinrichtung und/oder der Abführeinrichtung beweglich gegenüber einem drehbaren Träger angeordnet sind, insbesondere beweglich in einer Transportebene der Kunststoffbehältnisse. So könnten die einzelnen Halteeinrichtungen 36, 26 beispielsweise schwenkbar gegenüber dem drehbaren Träger angeordnet sein und/oder auch linear verschiebbar.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

1 Vorrichtung
2 Transporteinrichtung
4 erste Sterilisationseinrichtung
6 zweite Sterilisationseinrichtung
10 Kunststoffvorformling
10a Mündungsbereich
20 Abführeinrichtung
24 Träger
26 Halteeinrichtungen
28 Übergabebereich
34 Zuführeinrichtung
38 Übergabebereich
40 Reinraum
42 Ausführöffnung
44 Zuführöffnung
46 Wandungen
62 Austrittsfenster
64 Halteelemente
65 zweiter Gehäuseabschnitt
68 Schlitze
70 Grundkörper
100 Vorrichtung zum Sterilisieren von Behältnissen
104 Außensterilisationseinheit
106 Innensterilisationseinheit
112 Zuführeinrichtung
114 Abführeinrichtung

## Patentansprüche

1. Vorrichtung zum Sterilisieren von Kunststoffbehältnissen (10) mit einer Transporteinrichtung (2), welche die Kunststoffbehältnisse (10) entlang eines vorgegebenen Transportpfades transportiert, mit einer ersten Sterilisationseinrichtung (4), welche geeignet ist, eine Außenoberfläche der Kunststoffbehältnisse mit Strahlung zu beaufschlagen, um diese Außenoberfläche zu sterilisieren und mit einer zweiten Sterilisationseinrichtung (6), welche geeignet ist, eine Innenoberfläche der Kunststoffbehältnisse mit Strahlung zu beaufschlagen, um diese Innenoberfläche zu sterilisieren, wobei es sich bei der Strahlung um Ladungsträgerstrahlung handelt, wobei an der Transporteinrichtung (2) wenigstens eine Halteeinrichtung (8) angeordnet ist, welche ein Eingriffselement (60) aufweist, welches in einen Mündungsbereich des Kunststoffbehältnisses (10) einführbar ist, um diesen zu halten, **dadurch gekennzeichnet, dass** wenigstens ein Bestandteil der zweiten Sterilisationseinrichtung in die Halteinrichtung integriert ist und wobei die erste Sterilisationseinrichtung entlang eines Transportpfades der Halteeinrichtung (8) angeordnet ist, derart, dass die Kunststoffbehältnisse wenigstens zeitweise gleichzeitig von der ersten Sterilisationseinrichtung (4) und der zweiten Sterilisationseinrichtung (6) sterilisiert werden.

2. Vorrichtung (1) nach Anspruch 1,
**dadurch gekennzeichnet, dass**
die Vorrichtung eine Zuführeinrichtung (34) aufweist, welche der Halteeinrichtung (8) die Kunststoffbehältnisse (10) zuführt und diese Zuführeinrichtung oder die Transporteinrichtung (2) derart ausgeführt ist, dass sie einen Abstand zwischen zwei entlang des Transportpfades benachbarten Kunststoffbehältnissen verändern kann.

3. Vorrichtung (1) nach Anspruch 2,
**dadurch gekennzeichnet, dass**
die Transporteinrichtung und/oder die Zuführeinrichtung und/oder eine Abführeinrichtung die Kunststoffbehältnisse entlang Abschnitten des Transportpfades, welche unterschiedliche Krümmungsradien aufweisen, transportiert.

4. Vorrichtung (1) nach Anspruch 2 oder 3,
**dadurch gekennzeichnet, dass**
die Zuführeinrichtung und/oder die Abführeinrichtung wenigstens eine zweite Halteeinrichtung aufweist, welche geeignet ist, das Kunststoffbehältnisse an einem Außenbereich zu greifen.

5. Halteeinrichtung (8) zum Halten von Kunststoffbehältnissen (10) mit einem Eingriffselement (60), welches in einen Mündungsbereich des Kunststoffbehältnisses (10) einführbar ist, um diesen zu halten,
**dadurch gekennzeichnet, dass**
in diese Halteeinrichtung (8) eine Sterilisationseinrichtung (6) integriert ist, welche eine Innenoberfläche der Kunststoffbehältnisse von innen her beaufschlagt, wobei diese Sterilisationseinrichtung ein Austrittsfenster (62) aufweist, durch welches Ladungsträger austreten können, um einen Innenwandungsbereich der Kunststoffbehältnisse (10) zu sterilisieren.

6. Halteeinrichtung (8) nach Anspruch 5,
**dadurch gekennzeichnet, dass**
die Sterilisationseinrichtung ein Gehäuse (65) aufweist, innerhalb dessen Ladungsträger zu dem Austrittsfenster (62) gelangen.

7. Halteeinrichtung (8) nach Anspruch 6,
**dadurch gekennzeichnet, dass**
das Gehäuse (65) wenigstens abschnittsweise von dem Eingriffselement (60) umgeben ist.

8. Halteeinrichtung (8) nach Anspruch 5,
**dadurch gekennzeichnet, dass**
das Austrittsfenster in einer Längsrichtung (L) der Halteeinrichtung (8) gegenüber dem Eingriffselement (60) hervorsteht.

9. Verfahren zum Sterilisieren von Kunststoffbehältnissen (10), wobei die Kunststoffbehältnisse (10) mittels einer Transporteinrichtung (2) entlang eines vorgegebenen Transportpfades transportiert werden, und während dieses Transports wenigstens zeitweise eine Außenoberfläche der Kunststoffbehältnisse (10) von einer ersten Sterilisationseinrichtung (4) mit einer diese Außenoberfläche sterilisierenden Strahlung beaufschlagt wird, wobei es sich bei der Strahlung um Ladungsträgerstrahlung handelt,
**dadurch gekennzeichnet, dass**
die Kunststoffbehältnisse (10) mittels Halteeinrichtungen (8) transportiert werden, welche in einen Mündungsbereich der Kunststoffbehältnisse (10) eingeführt werden, und wobei wenigstens zeitweise während des Transports der Kunststoffbehältnisse (10)mittels der Transporteinrichtung (2) eine Innenoberfläche der Kunststoffbehältnisse (10) von einer zweiten Sterilisationseinrichtung (6) mit einer diese Innenoberfläche sterilisierenden Strahlung beaufschlagt wird und wenigstens zeitweise die Beaufschlagung der Innenoberfläche und die Beaufschlagung der Außenoberfläche gleichzeitig erfolgen.

10. Verfahren nach Anspruch 9,
**dadurch gekennzeichnet, dass**
während einer Zustellbewegung der Halteeinrichtung (8) auf die Kunststoffbehältnisse (10) zu und/oder während einer Wegführbewegung der Halteeinrichtung (8) von den Kunststoffbehältnissen (10) weg eine Sterilisation eines inneren Mündungsbereiches, insbesondere eines oberen Mündungsbereichs, der Kunststoffbehältnisse (10) vorgenommen wird.

11. Anlage mit einer Vorrichtung nach Anspruch 1,
**dadurch gekennzeichnet, dass**
die Vorrichtung zur Sterilisation der Kunststoffbehältnisse nach einer Erwärmungseinrichtung und/oder vor dem Ende einer Umformmaschine angeordnet ist.

## Claims

1. System for sterilising plastic containers (10) with a transport device (2) which transports the plastic containers (10) along a predetermined transport path, with a first sterilising device (4) which is suitable for applying radiation to an external surface of the plastic containers in order to sterilise this external surface, and with a second sterilising device (6) which is suitable for applying radiation to an internal surface of the plastic containers in order to sterilise this internal surface, wherein the radiation is a charge carrier radiation, wherein at least one holding device (8) is disposed on the transport device (2) and has an engaging element (60) which can be introduced into a mouth region of the plastic container (10) in order to hold it,
**characterized in that**
at least one component of the second sterilising device is integrated into the holding device, wherein the first sterilising device is disposed along a transport path of the holding device (8) in such a way that the plastic containers are at least intermittently sterilised simultaneously by the first sterilising device (4) and by the second sterilising device (6).

2. System (1) according to claim 1,
**characterized in that**
the system has a delivery device (34) which delivers the plastic containers (10) to the holding device (8) and this delivery device or the transport device (2) are designed in such a way that they can change a spacing between two adjacent plastic containers along the transport path.

3. System (1) according to claim 2,
**characterized in that**
the transport device and/or the delivery device and/or a discharge unit transports the plastic containers along portions of the transport path which have different radii of curvature.

4. System (1) according to claim 2 or 3,
**characterized in that**
the delivery device and/or the discharge unit has at least one second holding device which is suitable for gripping the plastic containers on an external region.

5. Holding device (8) for holding plastic containers (10) with an engaging element (60) which can be introduced into a mouth region of the plastic container (10) in order to hold it,
**characterized in that**
a sterilising device (6) is integrated into this holding device (8), which acts on an internal surface of the plastic containers from the inside, wherein this sterilising device has an exit window (62) through which charge carriers can exit in order to sterilise an inner wall region of the plastic containers (10).

6. Holding device (8) according to claim 5,
**characterized in that**
the sterilising device has a housing (65) inside which charge carriers reach the exit window (62).

7. Holding device (8) according to claim 6,
**characterized in that**
the housing (65) is surrounded at least in some sections by the engaging element (60).

8. Holding device (8) according to claim 5,
**characterized in that**
the exit window projects in a longitudinal direction (L) of the holding device (8) relative to the engaging element (60).

9. Method for sterilising plastic containers (10), wherein the plastic containers (10) are transported by means of a transport device (2) along a predetermined transport path, and during this transport at least intermittently an external surface of the plastic containers (10) is supplied by a first sterilising device (4) with radiation which sterilises this external surface, wherein the radiation is a charge carrier radiation,
**characterized in that**
the plastic containers (10) are transported by means of holding devices (8) which are introduced into plastic containers (10) in a mouth region thereof, wherein at least intermittently during the transport of the plastic containers by means of the transport device (2) an internal surface of the plastic containers (10) is supplied by a second sterilising device (6) with radiation which sterilises this internal surface and at least intermittently the supply to the internal surface and the supply to the external surface take place simultaneously.

10. Method according to claim 9,
**characterized in that**
during a feeding movement of the holding device (8) towards the plastic containers (10) and/or during a movement of the holding device (8) away from the plastic containers (10) sterilisation of the internal mouth region and in particular of an upper mouth region of the plastic containers (10) is carried out.

11. Installation according to claim 1,
**characterized in that**
the system for sterilising the plastic containers is disposed after a heating device and/or before the end of a transforming machine.

## Revendications

1. Dispositif de stérilisation de récipients en matière plastique (10), comprenant un système de transport (2), lequel transporte les récipients en matière plastique (10) le long d'un trajet de transport prédéfini, un premier système de stérilisation (4), lequel est adapté à exposer une surface extérieure des récipients en matière plastique à un rayonnement, afin de stériliser cette surface extérieure, et un deuxième système de stérilisation (6), lequel est adapté à exposer une surface intérieure des récipients en matière plastique à un rayonnement, afin de stériliser cette surface intérieure, dans lequel le rayonnement est un rayonnement porteur de charge, dans lequel au moins un système de retenue (8), lequel comprend un élément de mise en prise (60) pouvant être introduit dans une zone d'embouchure du récipient en matière plastique (10) afin de le retenir, est agencé sur le système de transport (2),
**caractérisé en ce qu'**
au moins un élément constitutif du deuxième système de stérilisation est intégré dans le système de retenue, et dans lequel le premier système de stérilisation est agencé le long d'un trajet de transport du système de retenue (8), de telle manière que les récipients en matière plastique sont stérilisés au moins temporairement simultanément par le premier système de stérilisation (4) et le deuxième système de stérilisation (6).

2. Dispositif (1) selon la revendication 1,
**caractérisé en ce que**
le dispositif comprend un système d'amenée (34), lequel amène les récipients en matière plastique (10) au système de retenue (8) et ce système d'amenée ou le système de transport (2) est réalisé de manière à modifier un écart entre deux récipients en matière plastique voisins le long du trajet de transport.

3. Dispositif (1) selon la revendication 2,
**caractérisé en ce que**
le système de transport et/ou le système d'amenée et/ou un système d'évacuation transporte les récipients en matière plastique le long de parties du trajet de transport, lesquelles présentent différents rayons de courbure.

4. Dispositif (1) selon la revendication 2 ou 3,
**caractérisé en ce que**
le système d'amenée et/ou le système d'évacuation comprennent au moins un deuxième système de retenue, lequel est adapté à saisir les récipients en matière plastique par une zone extérieure.

5. Système de retenue (8) destiné à retenir des récipients en matière plastique (10), comprenant un élément de mise en prise (60), lequel peut être introduit dans une zone d'embouchure du récipient en matière plastique (10), afin de le retenir,
**caractérisé en ce que**
ce système de retenue (8) intègre un système de stérilisation (6), lequel expose une surface intérieure des récipients en matière plastique depuis l'intérieur, dans lequel ce système de stérilisation comprend une fenêtre de sortie (62) par laquelle les porteurs de charge peuvent sortir, afin de stériliser une zone de paroi intérieure des récipients en matière plastique (10).

6. Système de retenue (8) selon la revendication 5,
**caractérisé en ce que**
le système de stérilisation comprend un logement (65) à l'intérieur duquel les porteurs de charge parviennent à la fenêtre de sortie (62).

7. Système de retenue (8) selon la revendication 6,
**caractérisé en ce que**
le logement (65) est entouré au moins en partie par l'élément de mise en prise (60).

8. Système de retenue (8) selon la revendication 5,
**caractérisé en ce que**
la fenêtre de sortie fait saillie dans une direction longitudinale (L) du système de retenue (8) par rapport à l'élément de mise en prise (60).

9. Procédé de stérilisation de récipients en matière plastique (10), dans lequel les récipients en matière plastique (10) sont transportés au moyen d'un système de transport (2) le long d'un trajet de transport prédéfini, et une surface extérieure des récipients en matière plastique (10) est sollicitée par un premier système de stérilisation (4) au moyen d'un rayonnement stérilisant cette surface extérieure au moins temporairement pendant ce transport, dans lequel le rayonnement est un rayonnement porteur de charge,
**caractérisé en ce que**
les récipients en matière plastique (10) sont transportés au moyen de systèmes de retenue (8), lesquels sont introduits dans une zone d'embouchure des récipients en matière plastique (10), et dans lequel une surface intérieure des récipients en matière plastique (10) est sollicitée par un deuxième système de stérilisation (6) au moyen d'un rayonnement stérilisant cette surface intérieure au moins temporairement pendant le transport des récipients en matière plastique (10) au moyen du système de transport (2) et l'exposition de la surface intérieure et l'exposition de la surface extérieure sont réalisées simultanément au moins temporairement.

10. Procédé selon la revendication 9,
**caractérisé en ce que**
une stérilisation d'une zone d'embouchure intérieure, en particulier d'une zone d'embouchure supérieure, des récipients en matière plastique (10) est réalisée pendant un mouvement de rapprochement du système de retenue (8) des récipients en matière plastique (10) et/ou pendant un mouvement d'éloignement des systèmes de retenue (8) des récipients en matière plastique (10).

11. Installation comprenant un dispositif selon la revendication 1,
**caractérisé en ce que**
le dispositif de stérilisation des récipients en matière plastique est agencé après un système de chauffage et/ou avant la fin d'une machine de mise en forme.
